# EUROPEAN PATENT SPECIFICATION

(11) **EP 3 010 590 B1**
(45) Date of publication and mention of the grant of the patent: **07.02.2018**
(21) Application number: 14738904.3
(22) Date of filing: 20.06.2014
(51) Int. Cl.: A61Q 5/00, A61K 8/19

(54) **DANDRUFF (AND SCALP) TREATMENT**
ANTISCHUPPEN (UND KOPFHAUT) BEHANDLUNG
TRAITEMENT DES PELLICULES DE CHEVEUX (ET DU CUIR CHEVELU)

(30) Priority: 21.06.2013 FR 1355948
(43) Date of publication of application: 27.04.2016
(73) Proprietor: L'OREAL, 75008 Paris (FR)
(72) Inventor: WOODLAND, Frédéric, F-93220 Gagny (FR); OSOLIN, Fabrice, F-95130 Franconville (FR); LEGENDRE, Jean-Yves, F-75013 Paris (FR); VIC, Gabin, 93400 Saint-Ouen (FR)
(74) Representative: Nony
(86) International application number: PCT/IB2014/062487
(87) International publication number: WO 2014/203218

(56) References cited:
- WO-A1-00/15168
- WO-A1-2004/105810
- WO-A2-2011/144344
- DE-A1-102011 003 533
- US-A1- 2013 068 226

## Description

The present invention is directed towards proposing a novel treatment for the prevention and/or treatment of disorders, in particular aesthetic disorders, of the scalp. In particular, the invention relates to dandruff disorders of the scalp.

The scalp is an epidermis that undergoes continual renewal, like the rest of the skin tissue, and that is rich in sebaceous glands.

Normally, the scalp undergoes renewal by removing the surface cells of the skin, without this renewal being felt or seen. However, excessive renewal of the cells of the *stratum corneum* of the scalp, for various reasons, may lead to the formation of large, thick patches of cells that are visible to the naked eye, known as "dandruff".

By way of example of factors which promote the appearance of dandruff, mention may be made of stress, the winter period, a lack of moisturisation or the colonisation of the skin or hair follicles by the yeast *Malassezia sp.* The *Malassezia sp.* genus consists of lipophilic yeasts normally present on the skin of human beings and of certain warm-blooded animals. Their distribution depends on age, on sebaceous gland activity, and on certain pathological conditions. The *Malassezia sp.* genus represents approximately 45% of the normal commensal flora at the surface of the scalp in dandruff-free individuals, but can represent 75% of the flora in the case of dandruff, and up to 85% in the case of associated seborrhoeic dermatitis. The other microorganisms generally present at the surface of the scalp are micrococci and *Propionobacterium.*

Dandruff conditions generally respond to various local or systemic antifungal treatments. Thus, various preparations combining antifungals and anti-seborrhoeic agents have been proposed in order to treat severe dandruff conditions. Antifungal-based treatments demonstrate a certain efficacy on dandruff conditions.

The use of a chemical antifungal integrated into a shampoo that will inhibit yeasts of *Malassezia* type at the surface of the scalp is relatively effective, but after use of the antidandruff shampoo is stopped, the dandruff reappears quite rapidly.

Moreover, numerous patent application publications relate to the use of plasmas in the medical field and the cosmetics field.

International application WO 2012/104332 discloses a deodorant treatment process by combining a cosmetic composition and a plasma.

WO 2012/103877 describes a process for treating the hair using a dielectric plasma.

WO 2004/105810 relates to a process for treating biological materials with an atmospheric plasma.

KR2006025825 describes an apparatus for dyeing the hair using an atmospheric cold plasma.

DE10238931 describes an apparatus for preparing a nail to receive a varnish.

CN101259036 describes a plasma generator in the form of a pencil, for treating the skin and removing blackheads.

DE102009060627 discloses an electrode arrangement for the dielectric barrier plasma treatment of a skin surface.

WO 2011/058301 describes a treatment apparatus used in cosmetics and in the dental field, using a cold plasma.

KR20090400156 discloses an anti-wrinkle plasma treatment apparatus.

WO 2011/144344 A2 discloses a device for at least partially sterilising a contaminated surface, using a cold plasma; various electrode arrangements for producing the plasma are described and a large number of applications are mentioned, for instance deodorising.

WO00/15168 teaches a topical antifungal composition for treating hair, skin and scalp for preventing dandruff.

There remains a need for novel solutions capable of exerting a beneficial action on scalp conditions, in particular of eliminating or reducing the appearance of dandruff.

The present invention meets this objective by virtue of a device according to claim 1.

The present specification also discloses the cosmetic use of a cold atmospheric plasma for preventing and/or treating aesthetic disorders of the healthy scalp, and in particular dandruff conditions of the healthy scalp.

The invention offers a solution which is effective right from the beginning of the treatment, and which is relatively long-lasting, rapid and easy to carry out.

Another advantage of the invention lies in the possibility of not calling upon any biocidal compound to be applied in the form of a lotion or shampoo for example.

For the purposes of the present invention, the term "preventing" means reducing the risk of manifestation of the phenomenon under consideration.

The present description also discloses a cosmetic process for treating and/or preventing aesthetic disorders of the healthy scalp in an individual, comprising the exposure of the scalp to a cold atmospheric plasma.

The disclosed process is in particular advantageously carried out in individuals exhibiting a dandruff condition of the scalp.

According to another of its aspects, a subject of the invention is also a device for use in treating the scalp, comprising:
- a member for producing cold atmospheric plasma, which makes it possible to expose the scalp to the plasma,
- a guide configured so as to bear against the scalp and to allow said member to move relative to the scalp at a predefined distance therefrom, in particular of between 0.5 and 15 mm, preferentially between 2 and 6 mm.

### Plasma

A plasma is an ionised gas which can also generate reactive species, produced by the discharge resulting in the plasma. A cold atmospheric plasma is a plasma which does not cause any excessive heating of the substrate exposed to the plasma.

Three families of cold atmospheric plasmas are available, namely direct, indirect and hybrid plasmas.

In direct plasmas, it is the substrate which acts as the counter electrode required for producing the plasma; the plasma is therefore generated between the electrode and the substrate. One of the main direct plasma technologies is the plasma known as DBD (Dielectric Barrier Discharge).

In indirect plasmas, the plasma is generated between two electrodes and is then directed towards the substrate using a gas stream. One of the main technologies is known as "plasma-torch".

Hybrid plasmas, known as corona plasmas, ally the mode of production of a direct plasma and the properties of indirect plasmas.

In the context of a direct or hybrid plasma, the scalp acts as the counter electrode and the area located under the electrode is treated.

Cold atmospheric plasmas make it possible to use various gases (air, helium, oxygen, etc). The invention is not limited in terms of gas. In direct plasma, the preferential gas is air.

In the context of the use of plasma alone, direct plasmas, and even more preferentially those of DBD type, are preferred in the invention.

### Treatment process

The process according to the invention comprises exposing the scalp to a cold atmospheric plasma.

The treatment area preferentially extends to the whole of the scalp.

It may then be necessary, in order to carry out the treatment, to move, relative to the scalp, a member which provides local production of the plasma.

The treatment time is adjusted according to the desired result.

Depending on the exposure time, the area of inhibition may be extended, thereby making it possible to treat a larger surface area than the surface area of the electrode or of the plasma source.

The treatment time is preferentially between 1" and 10', and more preferentially between 1" and 5'. This treatment time is entirely compatible with cosmetic use of a plasma generator for the treatment of dandruff conditions.

A 10" exposure already makes it possible to obtain an entirely satisfactory inhibition of *Malassezia* yeasts.

The plasma can be produced while maintaining the electrode or the source of plasma production at a controlled distance while it is moved at the surface of the scalp.

This movement can be carried out manually or in an automated manner. In the case of manual movement, the latter can be assisted, where appropriate, by a guidance structure placed around the person's head.

During the movement, the hair may be locally parted so as to facilitate access to the scalp, by means of a comb, for example.

The plasma production may be automatically interrupted over time, the generator stopping, for example, after a predefined operating period or when it is detected that the whole scalp has been treated.

The treatment may be repeated periodically, at intervals of 2 days to 1 month, for example.

When the treatment process comprises a topical application of a cosmetic composition, before, during or after exposure to the plasma, the penetration of the active agent(s) for treating the scalp may be facilitated, in particular owing to the fact that the plasma may decrease the lipid barrier function. The surface of the scalp may also be rendered more hydrophilic. The composition applied may in particular comprise one or more antidandruff active agents.

### Active agents

### Antidandruff active agents

The term "antidandruff active agent" is intended to mean a compound capable of preventing the appearance of dandruff, reducing the number of dandruff flakes and/or making said dandruff disappear entirely.

An antidandruff active agent that is suitable for use in the invention may be chosen in particular from:
- 1-hydroxy-2-pyridone derivatives, such as 1-hydroxy-4-methyl-2-pyridone, 1-hydroxy-6-methylpyridone, 1-hydroxy-4,6-dimethyl-2-pyridone, 1-hydroxy-4-methyl-6-(2,4,4-trimethylpentyl)-2-pyridone, 1-hydroxy-4-methyl-6-cyclohexyl-2-pyridone, 1-hydroxy-4-methyl-6-(methylcyclohexyl)2-pyridone, 1-hydroxy-4-methyl-6-(2-bicyclo[2,2,1]heptyl)-2-pyridone, 1-hydroxy-4-methyl-6(4-methylphenyl)-2-pyridone, 1-hydroxy-4-methyl-6[1-[4-nitrophenoxy]butyl]-2-pyridone, 1-hydroxy-4-methyl-6-(4-cyanophenoxymethyl-2-pyridone), 1-hydroxy-4-methyl-6-(phenylsulfonylmethyl)-2-pyridone, 1-hydroxy-4-methyl-6-(4-bromobenzyl)-2-pyridone and salts thereof; by way of preferred 1-hydroxy-2-pyridone derivative, mention may be made of the product sold by the company Hoechst under the name octopyrox (1-hydroxy-4-methyl-6-(2,4,4-trimethylpenthyl)-2-pyridone, monoethanolamine salt;
- pyridinethione salts, in particular the calcium, magnesium, barium, strontium, zinc, cadmium, tin and zirconium salts. The zinc salt of pyridinethione is particularly preferred. The zinc salt of pyridinethione is sold in particular under the name zinc omadine by the company Olin;
- trihalocarbamides of formula: 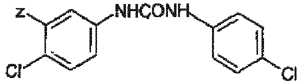 in which Z represents a halogen atom such as chlorine or a C₁-C₄ trihaloalkyl group such as CF₃;
- triclosan, represented by the formula: 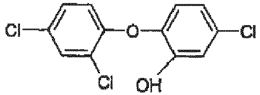
- azole compounds such as climbazole, ketoconazole, clotrimazole, econazole, isoconazole and miconazole;
- antifungal polymers such as amphotericin B or nystatin;
- selenium sulfides, in particular those of formula SₓSe₈₋ₓ, x ranging from 1 to 7;
- sulfur in its various forms, cadmium sulfide, allantoin, coal or wood tars and derivatives thereof, in particular cade oil, salicylic acid, undecylenic acid, fumaric acid, and allylamines such as terbinafine;
- ellagic acid;
- selenium disulfide.

### Treatment device

The plasma is generated by means of any suitable device which makes it possible to expose the scalp to a cold atmospheric plasma.

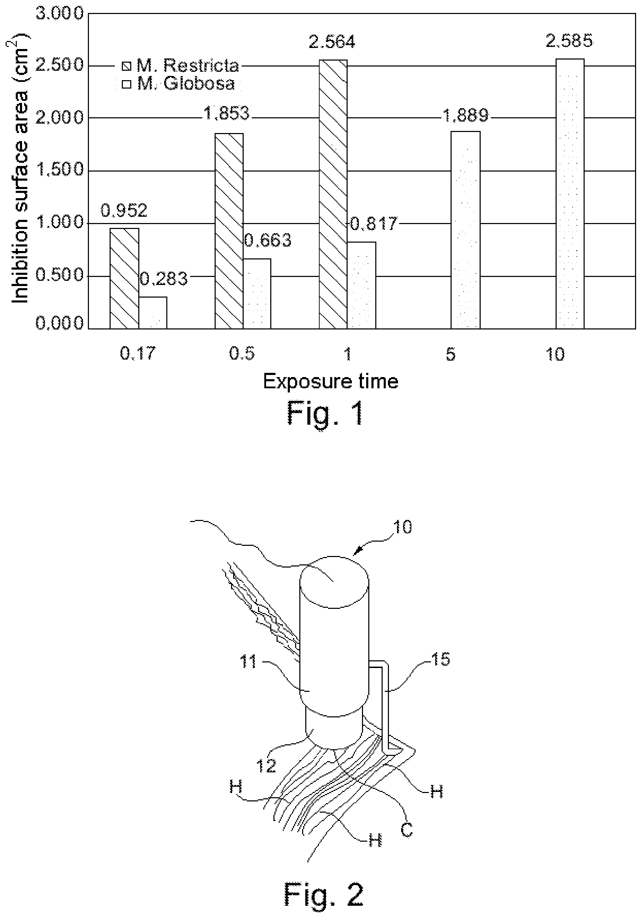

The emission of plasma is preferably carried out from a generator comprising a handpiece or in the form of a handpiece, the ergonomics of which are compatible with the application on the scalp. A Kinpen source sold by the company Neoplas tools GmbH, or a source in pen format as described in application CN101259036, or else a source in the form of a comb adapted for exposure of the scalp to the plasma, is for example used. An example of a device that can be used is described later, with reference to Figure 2.

Preferably, the device is arranged so as to provide uniform exposure of the scalp, by controlling the strength of the plasma produced.

The device may be arranged so as to maintain a predefined distance between the electrode or source of production of the plasma and the scalp, for example by virtue of a guide which bears against the scalp when the electrode or the source is moved relative to the scalp. This guide may have a curved surface which approximately moulds the curvature of the scalp.

The device may be arranged so as to locally part the hair in order to clear the scalp and facilitate the action of the plasma, and may comprise one or more comb teeth for this purpose.

The position of the device relative to the scalp when said device is moved may be detected, for example by means of accelerometers and/or of a camera, and the plasma production may be automatically controlled according to the position of the device so as to ensure the most complete and uniform precise treatment; for example, if it is detected that the device is again passing over a site on the scalp that has already been treated, the plasma production may be momentarily interrupted or reduced.

The device may be arranged so as to detect, for example by means of a camera, a movement of the device which is too fast to allow sufficient treatment, and to make the user aware of this.

The device may be mains-operated, rechargeable battery-operated and/or battery-operated.

The device may comprise a member for massaging the scalp, the action of which may or may not be concomitant with the plasma treatment.

### Tests

*In vitro* tests were carried out which demonstrate that a plasma treatment makes it possible to inhibit the growth of *Malassezia* yeasts from a relatively short exposure time onwards.

A growth medium (agar) was inoculated with a *Malassezia* culture.

The agar was then subjected to a plasma treatment of DBD type for a period ranging from 10" to 10'.

The inoculated dishes (3 per series of tests) were divided into quadrants and exposed to the plasma gas respectively for 10 s, 30 s and 60 s on three respective quadrants for the first series of tests, and 1 min, 5 min and 10 min for the second series of tests. The dishes were then incubated at 30-33°C for between 4 and 5 days.

At the end of the incubation period, all of the agar dishes were photographed.

The antifungal effect of the plasma gas was evaluated by measuring the surface area of inhibition of the *Malassezia* strain exposed, by means of the Cell F image analysis software (Olympus).

The surface area treated corresponds to the surface area of the electrode, i.e. approximately 1 cm².

### Results

Under the conditions of the test, the exposure to the plasma had an antifungal effect on the two strains tested (*Malessezia globosa* and *Malassezia restrict*)*.* The effect appears from as early as 10" of treatment and the surface area of inhibition around the area exposed increases as a function of exposure time, until it covers several cm².

### Malassezia restricta

| | *Malassezia restricta* (inhibition surface area in cm²) | | | |
|---|---|---|---|---|
| Treatment time | Test 1 | Test 2 | Test 3 | Mean |
| 10 s = 0.17 min | 0.612 | 0.879 | 1.366 | 0.952 |
| 30 s = 0.50 min | 1.752 | 1.624 | 2.183 | 1.853 |
| 1 min | 2.456 | 2.87 | 4.058 | 3.128 |
| | Mean of the two series for 1 minute: | | | 2.564 |
| 1 min | 2.03 | 1.770 | 2.193 | 2.000 |

### Malassezia globosa

| Time (min) | *Malassezia globosa* (inhibition surface area in cm²) | | | |
|---|---|---|---|---|
| Treatment time | Test 1 | Test 2 | Test 3 | Mean |
| 10 s = 0.17 min | 0.162 | 0.37 | 0.318 | 0.283 |
| 30 s = 0.5 min | 0.769 | 0.633 | 0.588 | 0.663 |
| 60 s = 1 min | 0.959 | 0.774 | 0.872 | 0.868 |
| | Mean of the two series for 1 minute: | | | 0.817 |
| 1 min | 0.699 | 0.836 | 0.763 | 0.766 |
| 5 min | 2.121 | 1.657 | 1.889 | 1.889 |
| 10 min | 2.423 | 2.765 | 2.566 | 2.585 |

Under the conditions of the tests, the *Malassezia restricta* strain is more sensitive than *Malassezia globosa.*

The results are reported in the appended Figure 1.

Figure 2 shows an example of a device 10 for treating the scalp according to the invention.

This device comprises a handpiece 11 to be moved on the scalp.

The plasma generator is, for example, of DBD type, and in this case, the handpiece 11 may comprise an electrode 12 covered with a dielectric material.

The electrode 12 is supplied with high-voltage pulses, in a manner known per se.

The scalp serves as a counter electrode, and receives a harmless electric current.

The device 10 comprises a guide 15 for maintaining the electrode 12 at a predefined distance from the scalp C. This guide 15 is also arranged so as to part the hair *H* upstream of the electrode 12, considering the direction of movement of the handpiece 11 relative to the scalp. This guide 15 may be in the form of a comb tooth with a rounded end piece, as illustrated.

The invention is not limited to the examples that have just been described.

In particular, the treatment may also be carried out differently, for example by being combined with the topical application of a cosmetic composition, in particular comprising an antidandruff active agent, or with the taking of a food supplement, before, during or after the plasma treatment.

## Claims

1. Device (10) for use in preventing and/or treating a dandruff condition of the scalp, comprising:
- a member (12) for producing a cold atmospheric plasma, which makes it possible to expose the scalp to the plasma,
- a guide (15) configured so as to bear against the scalp (C) and to allow said member to move relative to the scalp at a predefined distance therefrom, the distance being between 2 and 6 mm.

2. Device for use
according to Claim 1, the member for producing plasma belonging to a generator of direct type, preferably DBD type.

3. Device for use according to Claim 1, the plasma being of hybrid type.

4. Device for use according to Claim 1, the plasma being of indirect type.

5. Device for use
according to any one of the preceding claims, the gas used for the plasma production being air.

## Patentansprüche

1. Vorrichtung (19) zur Verwendung in der Vorbeugung gegen und/oder Behandlung von schuppiger Kopfhaut, umfassend:
- ein Bauteil (12) für die Erzeugung eines kalten Atmosphärendruckplasmas, das ermöglicht, die Kopfhaut dem Plasma auszusetzen,
- eine Führung (15), derart ausgebildet, dass sie gegen die Kopfhaut (C) drückt und ermöglicht, dass das Bauteil gegen die Kopfhaut in einem vordefinierten Abstand bewegt werden kann, wobei der Abstand zwischen 2 und 6 mm liegt.

2. Vorrichtung zur Verwendung gemäß Anspruch 1, wobei das Bauteil zur Herstellung des Plasmas zu einem Direktgenerator, vorzugsweise einem DBD-Generator gehört.

3. Vorrichtung zur Verwendung gemäß Anspruch 1, wobei das Plasma ein Hybrid-Plasma ist.

4. Vorrichtung zur Verwendung gemäß Anspruch 1, wobei das Plasma ein indirektes Plasma ist.

5. Vorrichtung zur Verwendung gemäß einem der vorangehenden Ansprüche, wobei das Gas, das für die Erzeugung des Plasmas genutzt wird, Luft ist.

## Revendications

1. Dispositif (10) destiné à être utilisé dans la prévention et/ou le traitement d'un état pelliculaire du cuir chevelu, comprenant :
- un organe (12) de production d'un plasma atmosphérique froid, permettant d'exposer le cuir chevelu au plasma,
- un guide (15) configuré pour prendre appui sur le cuir chevelu (C) et permettre de déplacer ledit organe relativement au cuir chevelu, à une distance prédéfinie de celui-ci, la distance étant comprise entre 2 et 6 mm.

2. Dispositif destiné à une utilisation selon la revendication 1, l'organe de production de plasma appartenant à un générateur de type direct, de préférence DBD.

3. Dispositif destiné à une utilisation selon la revendication 1, le plasma étant de type hybride.

4. Dispositif destiné à une utilisation selon la revendication 1, le plasma étant de type indirect.

5. Dispositif destiné à une utilisation selon l'une quelconque des revendications précédentes, le gaz utilisé pour la production du plasma étant de l'air.
